Europäisches Patentamt

**European Patent Office**

Office européen des brevets

(11) Numéro de publication : **0 082 745
B1**

# (12) FASCICULE DE BREVET EUROPÉEN

(45) Date de publication du fascicule du brevet :
**17.07.85**

(21) Numéro de dépôt : **82402197.6**

(22) Date de dépôt : **02.12.82**

(51) Int. Cl.⁴ : **A 61 K 49/00, C 12 P 1/00,
A 61 K 39/002// C12R1/90**

(54) **Nouvel antigène pour la recherche de l'immunité toxoplasmique et son procédé de préparation.**

(30) Priorité : **17.12.81 FR 8123565**

(43) Date de publication de la demande : .
**29.06.83 Bulletin 83/26**

(45) Mention de la délivrance du brevet :
**17.07.85 Bulletin 85/29**

(84) Etats contractants désignés :
**AT BE DE GB LU NL SE**

(56) Documents cités :
**LYON MEDICAL, vol. 243, no. 12, 30 juin 1980, pages 737-740, P.T. DESGEORGES et al.: "Mise en évidence et cinétique d'apparition d'exo-antigènes produits par toxoplasma gondii en culture-in vitro"
BIOLOGICAL ABSTRACTS, vol. 71, no. 12, décembre 1981, page 8510, no. 81144, Philadelphia, USA P.T. DESGEORGES et al.: "Modified gel electrophoresis derived enzyme linked immunosorbent assay test: Applications to Toxoplasma gondii exoantigens"
CHEMICAL ABSTRACTS, vol. 64, no. 13, 20 juin 1966, colonne 20386a, Columbus, Ohio, USA S.D. CHAPARAS et al.: "A laboratory procedure for determining the potency of toxoplasmins for skin testing"
CHEMICAL ABSTRACTS, vol. 62, no. 4, 15 février 1965, colonne 4369d-g, Columbus, Ohio, USA K. SHIMIZU: "Toxoplasmosis. III. Tissue culture of Toxoplasma gondii"**

(73) Titulaire : **INSTITUT MERIEUX (Société Anonyme)
27, rue Bourgelat B.P. 255
F-69223 Lyon Cedex 01 (FR)**

(72) Inventeur : **Roumiantzeff, Micha
Résidence de la Croix-Rousse 1 rue Dangon
F-69004 Lyon (FR)**
Inventeur : **Cagnard, Maryvonne
42 rue Alberic Pont
F-69005 Lyon (FR)**
Inventeur : **Ambroise Thomas, Pierre
2 Allée des Maronniers
F-38240 Meylan (FR)**

(74) Mandataire : **Lemoine, Michel et al
Cabinet Michel Lemoine 13 Boulevard des Batignolles
F-75008 Paris (FR)**

## 0 082 745

**Description**

La présente invention a trait à un nouvel antigène toxoplasmique pour la recherche de l'immunité toxoplasmique par tests, et à son procédé de préparation.

La toxoplasmose est une maladie à laquelle l'ensemble de la population tend à devenir de plus en plus sensible. Elle présente une gravité particulière pour le fœtus et la détection prénuptiale de l'immunité toxoplasmique a maintenant été rendue obligatoire.

La recherche de l'immunité peut être effectuée par des tests sérologiques mais, pour des raisons de simplicité et de coût, on a cherché à mettre au point des tests cutanés pour la détection de l'hypersensibilité retardée.

Déjà en 1948, J. K. Frenkel a mis au point des antigènes toxoplasmiques, désignés « toxoplasmins ». pour la détection de l'hypersensibilité retardée (Proceedings of the Society for Experimental Biology and Medicine 1948, 68, 634-639). Les antigènes étaient constitués par un broyat brut de parasites contenus dans l'exsudat péritonéal d'animaux tels que souris ou hamsters, du phénol à 0,25 % étant utilisé comme préservatif contre d'éventuelles contaminations du réactif au cours du stockage. Un réactif de ce genre a ensuite été mis sur le marché et a été largement utilisé (voir les publications de W. F. Mc Culloch — Public Health Reports 1963, 78, n° 8, 689-698 ; et de J. L. Krahenbuhl — Infection and Immunity 1971, 3, n° 2, 260-267). Une telle préparation ne satisfait cependant pas aux normes actuelles d'innocuité à cause de la présence éventuelle de contaminants de l'exsudat de souris.

On a ensuite essayé d'obtenir des préparations d'antigènes par culture de Toxoplasma gondii sur des lignées de cellules de reins de singe. L'antigène était obtenu par congélation et décongélation des parasites recueillis (voir S. D. Chaparas et al. — Proceedings of the Society for Experimental Biology and Medicine 1966, 121, 734-739). Ces préparations antigéniques utilisées par intradermoréaction à la seringue, comme le réactif mis au point à partir des travaux de J. K. Frenkel, présentent cependant l'inconvénient de contenir de nombreuses particules provenant des parasites.

On a ensuite pu mettre en évidence, dans des cultures sur des lignées de cellules diploïdes d'origine humaine ou sur des lignées de cellules de reins de singe, que les toxoplasmes libèrent dans le milieu de culture des exo-antigènes (P. T. Desgeorges, X. Billiault, P. Ambroise-Thomas, et M. Bouttaz, Lyon Médical 30, 06, 80-243, 12, 737-740). On a ainsi pu montrer qu'il devenait envisageable d'utiliser de tels exo-antigènes toxoplasmiques pour la recherche de l'immunité toxoplasmique. Ces antigènes présentent en effet l'avantage d'être pratiquement exempts de matériaux particulaires provenant des toxoplasmes, et de ne pas contenir de contaminant d'origine cellulaire. Néanmoins, les préparations antigéniques ainsi décrites ne sont pas adaptées à une utilisation dans le domaine humain.

L'objectif de l'invention est donc de fournir un tel antigène toxoplasmique, pour la recherche de l'immunité toxoplasmique, qui présente un grand degré de pureté et qui soit dépourvu de contaminants à haut risque de sensibilisation pour les humains.

Un autre objectif de l'invention est de fournir un tel antigène qui permette la recherche de l'immunité toxoplasmique par tests cutanés par multipuncture.

Un autre objectif de l'invention est de fournir un tel antigène qui soit également susceptible d'être utilisé dans des tests sérologiques.

Un autre objectif encore de l'invention est de fournir un tel antigène ne présentant pas de risques de contamination virale.

L'invention a pour objet un antigène toxoplasmique, pour la détection de l'immunité toxoplasmique chez l'homme, caractérisé en ce qu'il est composé d'exo-antigène exempt de débris pariétaux et de sérum, ledit antigène ayant été concentré à un titre d'au moins 500 unités toxoplasmiques HSR Mérieux d'hypersensibilité sur cobaye, et inactivé.

Une telle unité HSR est définie comme la quantité d'antigène qui, injectée par voie intradermique sous un volume de 0,1 ml à un cobaye spécifiquement sensibilisé, donne une réaction de 100 mm$^2$ en 18 heures ou 24 heures.

De préférence, l'antigène présente une concentration de l'ordre de deux cents fois à partir de la récolte initiale.

L'invention a également pour objet un procédé de préparation d'un antigène toxoplasmique pour la recherche de l'immunité toxoplasmique, dans lequel on inocule une culture de cellules dans un milieu approprié, à l'aide de Toxoplasma gondii, et l'on recueille le milieu de culture, dépourvu de cellules et de toxoplasmes et contenant des exo-antigènes toxoplasmiques, caractérisé en ce que l'on effectue la culture en présence de toxoplasmes dans un milieu dépourvu de sérum animal, tel que notamment le sérum de veau, que l'on élimine les toxoplasmes et les débris pariétaux en provenance des cellules ou des toxoplasmes par filtration stérilisante, que l'on traite l'antigène soluble obtenu après filtration par un processus d'inactivation des particules virales éventuellement présentes, de préférence au formol, et que l'on concentre l'antigène soluble après inactivation.

La culture de cellules peut être une culture de cellules primaires de différentes espèces, telles que cellules de reins de singe, de porc, cellules de poulet mais, de préférence, on utilise soit des lignées humaines diploïdes, soit des lignées animales hétéroploïdes.

De préférence on utilise, comme lignée de cellules diploïdes humaines. la lignée cellulaire MRC 5. Cette lignée est décrite par J. P. Jacobs et al. — Nature 1970, 227, n° 5254, 168-170 et par exemple

2

disponible auprès de National Institute for Medical Research Holly Hill — London NW3 — Grande-Bretagne.

Comme lignée de cellules animales hétéroploïdes, on utilise de préférence des lignées non tumorigènes telles que la lignée VERO décrite par B. J. Montagnon et coll. Intern. Symp. on Reassessment of Inactivated Poliomyelitis Vaccine, Bilthoven 1980, Develop. Biol. Standard, 47, p. 55-64 (S. Karger, Basel 1981).

Dans un mode de réalisation particulièrement préféré, on effectue une concentration suffisante, au moins de 190 fois et de préférence d'environ deux cents fois, pour obtenir un antigène titrant au minimum 160 unités au test ELISA, ce qui correspond sensiblement à 500 unités HSR Mérieux, puis l'on met l'antigène en suspension dans du glycérol pour pouvoir l'utiliser par la méthode de scarification, notamment par multipuncture.

Le titre ELISA, pour un antigène, est le $\log_{10}$ de l'inverse de la dilution à 50 % de la densité optique maximale estimée sur la plaque en excès d'antigène. L'activité de l'antigène inconnu est exprimée par rapport à celle d'un antigène standard titré dans les mêmes conditions. On calcule une puissance relative égale à l'antilog de la différence du titre ELISA de l'antigène inconnu avec celui de l'antigène standard. Le titre final de l'antigène à tester est donné par le produit de sa puissance relative par le titre du standard en unités par millilitre.

De façon avantageuse, la culture cellulaire après inoculation est poursuivie environ six jours à 37 °C.

L'étape de filtration peut avantageusement comporter une première filtration stérilisante, suivie d'une dialyse ultra-filtrante, puis d'une nouvelle filtration stérilisante.

L'inactivation est effectuée de préférence au formol à un pour mille pendant environ vingt-quatre heures à température ambiante sous agitation permanente.

Selon un mode de réalisation préféré, l'antigène ainsi concentré est mis en suspension dans du glycérol ou un autre vecteur, pour être utilisé par une méthode de scarification, notamment par multipuncture.

D'autres avantages et caractéristiques de l'invention apparaîtront à la lecture de la description suivante, faite à titre d'exemple non limitatif.

On effectue une culture de cellules MRC 5 dans un milieu EAGLE BME additionné de 10 % de sérum de veau. Cette culture est effectuée dans des flacons de 500 cm² de surface environ.

La culture s'effectue à 37 °C pendant une durée de 3 jours avec une concentration initiale de cellules de 20 millions par flacon dans du milieu EAGLE BME, plus 10 % de sérum de veau. Au troisième jour, le milieu est remplacé par du milieu EAGLE BME additionné de 2 % de sérum de veau. Les flacons sont remis à 37 °C pendant 4 jours. Ensuite, après élimination du milieu, on procède à 3 lavages du tapis cellulaire par de l'eau physiologique et on remet les cellules en milieu EAGLE BME dépourvu de sérum de veau. Vingt-quatre heures après ces lavages, on effectue un nouveau changement de milieu BME sans sérum de veau avant l'introduction des parasites. Les toxoplasmes entretenus sur souris femelle type Swiss sont inoculés à raison de 2,4 ‰ $10^5$ toxoplasmes par ml de milieu de culture.

On effectue alors une culture pendant six jours à 37 °C.

Après le sixième jour, le surnageant de la culture est récolté. Celui-ci subit une première filtration stérilisante (préfiltre AP 20 — AW 06 — filtres 0,22 µm stériles Millipore®).

Ensuite on procède à l'adjonction de formol à un pour mille et la solution est maintenue à la température ambiante sous agitation permanente pendant vingt-quatre heures. Cette phase d'inactivation est suivie d'une dialyse ultrafiltrante et d'une concentration d'au moins 200 fois avec du matériel (colonne et cellules) Amicon sur membrane PM 10.

Le concentrat subit ensuite une refiltration stérilisante sur filtres Millipore® successifs (AP 20, 8 µm ... 0,45 µm, 0,22 µm).

A ce stade, la solution d'antigène obtenue présente un titre en hypersensibilité retardée sur cobaye d'au moins 500 ± 20 % unités toxoplasmiques HSR Mérieux.

L'antigène soluble purifié, concentré et inactivé est ensuite titré sérologiquement in vitro par un test ELISA, puis calibré pour son activité d'hypersensibilité retardée à l'aide d'un titrage in vivo sur des cobayes préalablement sensibilisés par un extrait particulaire de Toxoplasma gondii.

Enfin, l'antigène est mis en suspension dans du glycérol à 70 %, la préparation étant destinée à être appliquée sur les pointes d'un dispositif de multipuncture.

Les études précliniques ont permis de donner les résultats suivants :

Sur cobayes

| Dilution Antigènes | pur | 1/3e | 1/10e | 1/30e | 1/100e |
|---|---|---|---|---|---|
| Lot X | 5,5 mm | 4,2 | 2,5 | 0 | 0 |
| Lot Y | 5,8 mm | 4,2 | 3,2 | 2,8 | 1 |

Sur humains

| T.L. | Sér.<br>p.i. | Antigène X pur | | | Antigène X 1/3 | | | Antigène X 1/10e | | |
|---|---|---|---|---|---|---|---|---|---|---|
| | | 24 h | 48 h | 72 h | 24 h | 48 h | 72 h | 24 h | 48 h | 72 h |
| Sujet A | 160 u.i. | 4,5 min | 4,5 min | 3,5 min | 0 | 3 min | 3 min | 2 min | 2 min | 2 min |
| Sujet B | 80 u.i. | 0 | 3 min | 3,5 min | 0 | 0 min | 0 min | 0 min | 0 min | 0 min |
| Sujet C | 8 u.i. | 0 | 2 min | 2,5 min | — | — | — | — | — | — |
| Sujet D | 0 | 0 | 0 | 0 | 0 | 0 | 0 | 0 | 0 | 0 |

Légende des abréviations du tableau précédent.
— Sér. : Sérologie ;
— T.L. : temps de lecture ;
— p.i. : par immunofluorescence.

**Revendications**

1. Antigène toxoplasmique, pour la détection de l'immunité toxoplasmique chez l'homme, caractérisé en ce qu'il est composé d'exo-antigène exempt de débris pariétaux et de sérum, ledit antigène ayant été concentré à un titre d'au moins 500 unités toxoplasmiques HSR Mérieux d'hypersensibilité sur cobaye, et inactivé.

2. Antigène toxoplasmique selon la revendication 1, caractérisé en ce qu'il est mis en suspension dans un vecteur tel que du glycérol pour être utilisé par une méthode de multipuncture.

3. Procédé de préparation d'un antigène toxoplasmique pour la détection de l'immunité toxoplasmique dans lequel on inocule une culture de cellules dans un milieu approprié, à l'aide de Toxoplasma gondii, et l'on recueille le milieu de culture, dépourvu de cellules et de toxoplasmes et contenant des exo-antigènes toxoplasmiques, caractérisé en ce qu'on effectue la culture en présence de toxoplasmes dans un milieu dépourvu de sérum animal tel que notamment le sérum de veau, que l'on élimine les toxoplasmes et les débris pariétaux en provenance des cellules ou des toxoplasmes par filtration stérilisante, que l'on concentre l'antigène soluble obtenu après filtration, et que l'on traite l'antigène par un processus d'inactivation des particules virales.

4. Procédé selon la revendication 3, caractérisé en ce que l'on effectue une concentration d'au moins deux cents fois.

5. Procédé selon l'une quelconque des revendications 3 et 4, caractérisé en ce qu'on utilise une lignée de cellules diploïdes humaines et notamment la lignée cellulaire MRC 5.

6. Procédé selon l'une quelconque des revendications 3 et 4, caractérisé en ce que l'on utilise une lignée de cellules animales hétéroploïdes et notamment la lignée VERO.

7. Procédé selon l'une quelconque des revendications 3 et 4, caractérisé en ce que l'on utilise une culture de cellules primaires.

8. Procédé selon l'une quelconque des revendications 3 à 7, caractérisé en ce que l'on met l'antigène en suspension dans du glycérol.

9. Procédé selon l'une quelconque des revendications 3 à 8, caractérisé en ce qu'on effectue la culture cellulaire après inoculation pendant six jours à 37 °C.

10. Procédé selon l'une quelconque des revendications 3 à 9, caractérisé en ce que la filtration comporte une étape de filtration stérilisante suivie d'une dialyse ultra-filtrante, puis d'une nouvelle filtration stérilisante.

11. Procédé selon l'une quelconque des revendications 3 à 10, caractérisé en ce que l'on effectue l'inactivation au formol.

12. Procédé selon la revendication 11, caractérisé en ce que l'inactivation au formol est effectuée pendant environ vingt-quatre heures au formol à un pour mille à température ambiante.

**Claims**

1. A toxoplasmic antigen, for the detection of toxoplasmic immunity in man, characterised in that it is composed of exo-antigen free from parietal waste and from serum, the said antigen having been concentrated to a figure of at least 500 HSR Merieux toxoplasmic units of hypersensitivity on guinea pig, and inactivated.

**0 082 745**

2. A toxoplasmic antigen according to claim 1, characterised in that it is put in suspension in a vector such as glycerol in order to be used by a multipuncture method.

3. A process for the preparation of a toxoplasmic antigen for the detection of the toxoplasmic immunity which one innoculates a culture of cells in an appropriate medium, with the aid of gondii Toxoplasma, and one harvests the culture medium, lacking in cells and toxoplasms and containing toxoplasmic exo-antigens, characterised in that one carries out the culture in the presence of toxoplasms in a medium lacking in animal serum such as notably serum of calf, that one eliminates the toxoplasms and parietal debris deriving from the cells or from the toxoplasms by sterilizing filtration, that one concentrates the soluable antigen obtained after filtration, and that one treats the antigen by a process of inactivation of viral particles.

4. A process according to claim 3, characterised in that one carries out a concentration of at least 200 times.

5. A process according to either of claims 3 and 4, characterised in that one uses a line of diploid human cells and notably the MRC 5 cellular line.

6. A process according to either of claims 3 and 4, characterised in that one uses a line of heteroploid animal cells and particularly the VERO line.

7. A process according to either of claims 3 or 4, characterised in that one uses a culture of primary cells.

8. A process according to any of claims 3 to 7, characterised in that one puts the antigen in suspension in glycerol.

9. A process according to any of claims 3 to 8, characterised in that one effects the cellular culture after inoculation for six days at 37 °C.

10. A process according to any of claims 3 to 9, characterised in that the filtration comprises a step of sterilizing filtration followed by an ultrafiltering dialysis, and then by a new sterilizing filtration.

11. A process according to any of claims 3 to 10, characterised in that one carries out the inactivation with formol.

12. A process according to claim 11, characterised in that the inactivation with formol is effected during about 24 hours in formol at 1 per thousand at ambient temperature.

**Patentansprüche**

1. Toxoplasmisches Antigen für die Ermittlung der toxoplasmischen Immunität am Menschen, dadurch gekennzeichnet, dass es aus einem von Wandungsteilchen und von Serum freien Exo-Antigen zusammengesetzt ist, welches Antigen auf einen Gehalt von mindestens 500 toxoplasmischen HSR-Mérieux-Einheiten der Hypersensibilität am Meerschweinchen konzentriert und inaktiviert ist.

2. Toxoplasmisches Antigen nach Anspruch 1, dadurch gekennzeichnet, dass es für die Anwendung durch eine Vielfachpunktionmethode in einem Träger, wie Glyzerin, suspendiert ist.

3. Verfahren zur Herstellung eines toxoplasmischen Antigens für die Ermittlung der toxoplasmischen Immunität, bei dem man eine Zellkultur in ein entsprechendes Milieu mit Hilfe von Toxoplasma gondii inokuliert und das von Zellen und Toxoplasmen freie, und toxoplasmische Exo-Antigen enthaltende Kulturmilieu aufsammelt, dadurch gekennzeichnet, dass man die Kultur in Gegenwart von Toxoplasmen in einem von tierischem Serum, wie insbesondere Kalbserum, freien Milieu aufzieht, dass man die Toxoplasmen und die von den Zellen oder Toxoplasmen herrührenden Wandungsteilchen durch Sterilisationsfiltration beseitigt, dass man das nach dem Filtern erhaltene lösliche Antigen konzentriert, und dass man das Antigen einer Behandlung zur Inaktivierung von Virusteilchen unterzieht.

4. Verfahren nach Anspruch 3, dadurch gekennzeichnet, dass man eine zumindest zweihundertfache Konzentration vornimmt.

5. Verfahren nach einem der Ansprüche 3 oder 4, dadurch gekennzeichnet, dass man diploïde menschliche Zellstämme und insbesondere den Zellstamm MRC 5 verwendet.

6. Verfahren nach einem der Ansprüche 3 oder 4, dadurch gekennzeichnet, dass man einen heteroploïden Zellstamm und insbesondere den Stamm VERO verwendet.

7. Verfahren nach einem der Ansprüche 3 oder 4, dadurch gekennzeichnet, dass man eine Kultur von Primärzellen verwendet.

8. Verfahren nach einem der Ansprüche 3 bis 7, dadurch gekennzeichnet, dass man das Antigen in Glyzerin suspendiert.

9. Verfahren nach einem der Ansprüche 3 bis 8, dadurch gekennzeichnet, dass man die Kultur nach dem Inokulieren während sechs Tagen bei 37 °C belässt.

10. Verfahren nach einem der Ansprüche 3 bis 9, dadurch gekennzeichnet, dass der Filtriervorgang eine Sterilisationsfiltrationsstufe aufweist, die von einer ultrafiltrierenden Dialyse und sodann von einer neuerlichen Sterilisationsfiltration gefolgt ist.

11. Verfahren nach einem der Ansprüche 3 bis 10, dadurch gekennzeichnet, dass man die Inaktivierung mit Formaldehyd vornimmt.

12. Verfahren nach Anspruch 11, dadurch gekennzeichnet, dass man die Inaktivierung mit Formaldehyd während etwa vierundzwanzig Stunden mit einem Promille Formaldehyd bei Umgebungstemperatur vornimmt.